# EUROPEAN PATENT APPLICATION

(11) **EP 0 768 062 A1**
(43) Date of publication of application: **16.04.1997**
(21) Application number: 96307370.5
(22) Date of filing: 10.10.1996
(51) Int. Cl.: A61B 17/064, A61B 17/68

(54) **A plate-staple for use in osteotomy on proximal portion of tibia**

(30) Priority: 11.10.1995 KR 9534800
(71) Applicant: Bae, Dae-Kyung, Kangnam-gu, Seoul (KR)
(72) Inventor: Bae, Dae-Kyung, Kangnam-gu, Seoul (KR)
(74) Representative: Jenkins, Peter David

(57) **Abstract**

The present invention relates to a plate-staple (1) comprising a fixing pin (2) having a pair of legs with each leg terminating in a sharp point, a bridging member (4) for interconnecting the two legs to form a stepped U-shaped staple having one leg longer relative to the other leg, and a plate (3) having a pair of holes (6) bored therein which each in use receive a screw (9) and a top recess (7) formed thereon such that in use the bridging member (4) is received by the recess (7). The plate-staple (1) of the present invention does not require plaster fixing and articulation is possible immediately after the operation. Walking with a crutch is possible about three weeks after the operation, and no re-hospitalization for rehabilitation therapy is necessary.

## Description

The present invention relates to a plate-staple and, more particularly, to a mini-plate and staple for use in osteotomy on proximal portion of tibia which provides holding power more than 6 times stronger than that of conventional staple.

In the area of orthopaedics, osteoarthritis of the knee joint, a bow-leg and other deformation of the lower limb due to trauma or congenital disease are remedied by performing a corrective operation. A staple, which is an internal fixing piece, is used in fixing the corrected portion until bones are joined after the corrective operation (i.e. osteotomy). Such staple can be used in the fixing bone and in the correction of a difference in the length of the lower limbs at the time of remedying a trauma such as bone fracture or dislocation of bone to different portions of body in the area of orthopaedics.

In the prior art, proximal portions of the tibia are fixed by drilling holes at portions to be simply combined or supported and inserting a staple to support the two portions, however, a staple is of a structure which can not mechanically fully support the two portions.

For such prior art staple, a coventry staple devised by coventry of USA in 1960 has been used and in addition, sometimes an angle blade plate has been used, However, the coventry staple has a defect in that it does not provide sufficient holding power so that after the operation it slips out from the portion where it was fixed or the correction portion moves. The angle blade has disadvantages in that it has a relatively large staple so that the operation must be extensive, in that the corrected portion must be relatively low for the insertion of the angle blade so that the joining of the bones is delayed, and since the blade plate has a constant angle so that the correction portion frequently moves when the blade plate is internally fixed, it is difficult to maintain the correction angle. Various methods of remedy have been used to correct, and until very recently this method has been mostly used in which osteotomy is applied to the proximal portion of tibia, then the tibia is fixed using fixing pins, and then the tibia is plaster fixed until bones are joined. However such method of remedy has a disadvantage in that it gives annoyance of plaster fixing for about six weeks after the operation and re-hospitalization is necessary for rehabilitative therapy after removal of the plaster fixture and long term physical therapy is required.

The present invention solves the above described problems, and is composed of a fixing pin portion and a small metal plate. The purpose of the present invention is to provide a mini-plate staple which can be easily inserted into a bone since the fixing pin is of circular structure and has sharp ends and which provide a strong fixing force.

In the present invention, the upper and lower parts of the staple have dimensions appropriate to the Oriental skeleton, and a small metal plate is attached to the lower part of the staple. Two screw holes are in the metal plate so that a fixing screw can be inserted through the hole. The size of the screw hole is such that the screw can be fixedly as inclined in any direction, for example, upward, downward, left and right direction. This is made possible by having the diameter of the screw hole being somewhat larger than the diameter of the body of the screw and smaller than the diameter of the head of screw. The fixing force of the inserted fixing pin can be greatly increased by fixing the small metal plate and two screws, the deformation of lower limbs can be remedied without change of the correction angle and the operation time and the operation can be satisfactorily performed with a simple and convenient operation. This must be the first throughout the world since there is no report about a mini-plate staple in which the metal plate is attached to the fixing pin.

The mini-plate staple is composed of a fixing pin portion and a small metal plate. The upper and lower fixing pins are of circular construction and have sharp ends so as to be easily inserted into the bone. The small metal plate is secured, in use, to a portion of the bridging member of the fixing pin. The metal plate includes two screw receiving holes bored therein. After the osteotomy on the proximal portion of tibia, a fixing pin is inserted to be fixed at upper and lower part of the portion on which the osteotomy is performed, then it is confirmed whether the fixing pin is at appropriate position by X-ray, or the like. Then, holes are drilled by using a drilling machine to insert a screw, the length of hole is measured, then the fixing pin is fixed by using a screw having the same length as the hole. Two screws are inserted in the same way, and then the presence of the abnormality is confirmed by X-ray.

Although the fixing force of the conventional product is produced by only the fixing pin, the mini-plate staple of the present invention uses includes two screws to secure the small metal plate so that a total fixing force is produced by the fixing pin and the plate against the fixing pin. It is important to point out that the fixing pin is positioned over the plate, i.e. the plate is not positioned over the fixing pin so as to force the pin against the bone. Notwithstanding this positioning, when tested, the holding strength of the mini-plate staple according to the present invention appeared to be about 6 times stronger than the conventional staple. In addition an advantage of the present invention is that since a little amount of osteolysis appears around the fixing pin after operation when used in the human bone, the mini-plate staple of the present invention never slips out or moves after insertion. This aspect is very different from the conventional product in which only the fixing pin is used.

A preferred embodiment of the present invention will now be described hereinbelow by way of example only with reference to the accompanying drawings, in which:
Fig. 1 is a side view of the prior art staple;
Fig. 2 is a side view of the plate-staple of the present invention;
Fig. 3 is a side view showing the needle part of the plate-staple of the present invention together with the plate in broken-lines;
Fig. 4 is a plan view showing only the plate of the mini plate-staple of the present invention;
Fig. 5 is a side view showing only the plate of the mini plate-staple of the present invention; and
Fig. 6 is a perspective view of the plate-staple of the present invention operatively positioned in use.

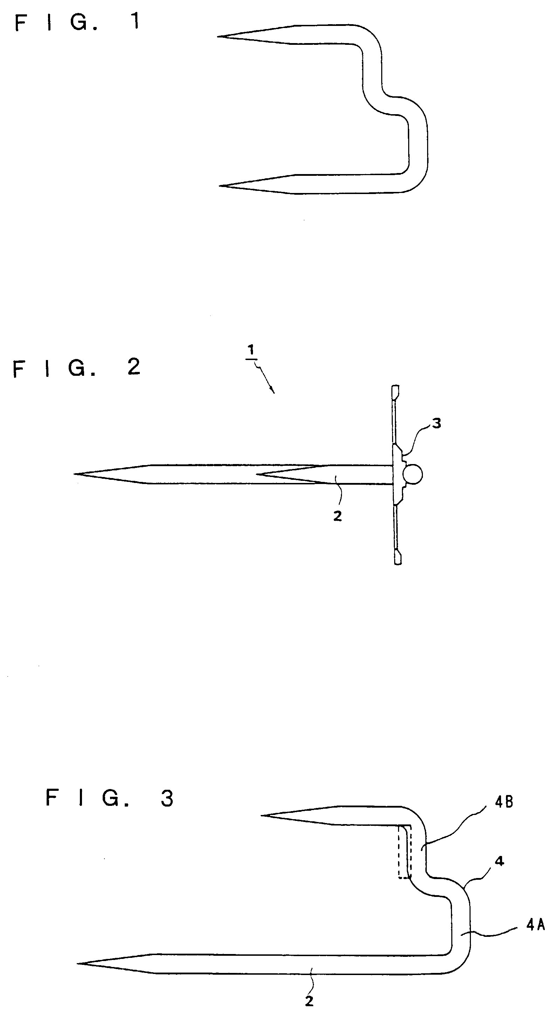

Fig. 1 is a side view of the prior art fixing pin or staple illustrating each leg being of the same length.

Fig. 2 is a side view of the combination plate-staple 1 according to the present invention. The plate-staple 1 is composed of a fixing pin 2 and a small metal plate 3.

Fig. 3 is a side view of the fixing pin 2. The fixing pin 2 has sharp ends and two legs. One leg is long and the other leg is short. The bridging member 4 interconnects the pair of legs, to define a stepped U-shaped staple, i.e. an offset portion and an aligned (perpendicular to the legs) portion with the aligned portion being received in the recess of the plate. The position of the mini-plate, in use, is shown in broken lines. Preferably, the bridging member 4 includes an offset portion 4A and an aligned portion 4B. The offset portion 4A bridges the defect when the staple is positioned in use. The aligned portion 4B is received into the recess 7 formed on the top of the plate 1, shown in broken lines.

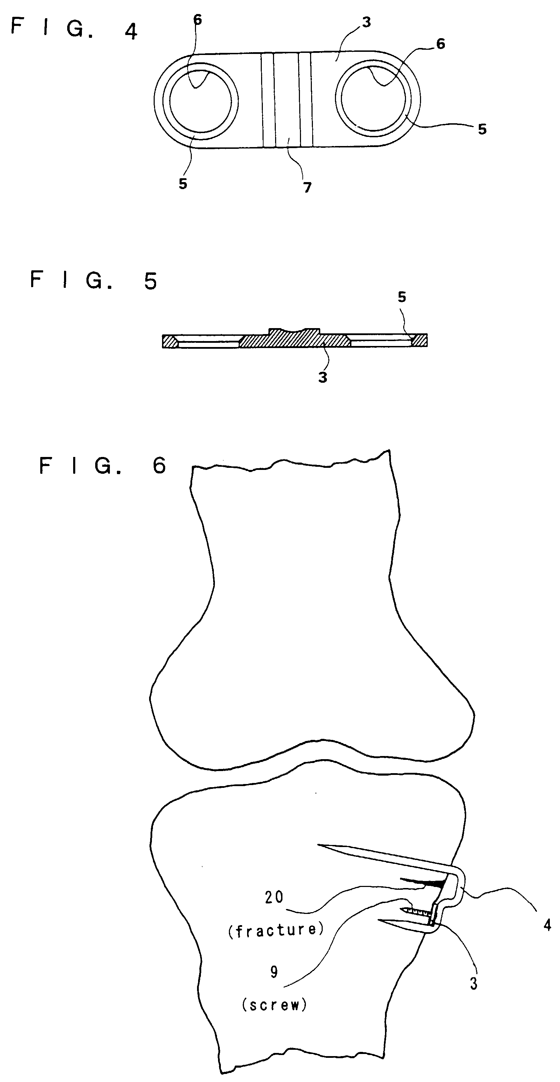

Figs. 4 and 5 are, respectively, a plan view and a side view of the small metal plate 3. The small metal plate 3 has two holes 6 each having a counter sink 5, with one hole 6 on each side. A recess 7 is formed at the center of the small metal plate 3 for receiving therein a portion of the stepped bridging member 4, as see Fig. 3. Hole 6 formed in the metal plate 3 is for receiving therethrough a screw (not shown).

Fig. 6 illustrates the mini-plate staple of the present invention operatively positioned in use to correct deformities resulting from conditions, such as, degenerative arthritis, rheumatoid arthritis or trauma. As is readily apparent from Fig. 6, the mini-plate 3 is positioned in the bone and then the staple is positioned with the offset portion 4A of the bridging part 4 of the staple of the present invention bridging the deformity 20 (e.g. a fracture) and a portion of the aligned portion 4B received into recess 7 of the mini-plate 3. The mini-plate is secured into the bone via screws 9 received into each hole 6 bored into the mini-plate 3. Surprisingly, even though the staple of the present invention is only positioned in recess 7 of the mini-plate, the holding power of the mini-plate staple of the present invention, with one leg being longer relative to the other leg, is six (6) times greater than that of the staple alone.

Reviewing the sequence of the operation with the mini-plate state 1 constructed as described above:
(1) An osteotomy is performed on the proximal portion of tibia at the time of operation, then the fixing pin is inserted into the portion where the osteotomy is performed, in such a way that the fixing pin is fixed on the upper part and the lower part of the portion, and then it is confirmed by radiation ray photographing whether the fixing pin is in the proper position.
(2) A hole is drilled using a drilling machine for inserting a screw therein, then the length of the hole is measured, and then the fixing machine is fixed by utilizing a screw having the same length as the hole.
(3) Two screws are inserted in the same way and then the presence of abnormality is confirmed by the radiation ray photographing.

The results of testing the use of the mini-plate staple of the present invention and the staple of the prior art in an animal is described below.

### 1. Test material

### (1) Tibia used in the test

An animal used in the test was a pig (Yorkshire species) 5 months old, weighing over 90kg and having experienced no disease. For use in the test, the pig was slaughtered at a slaughterhouse, quickly transported to a bone bank and kept there until the time of test in a refrigerating room below -72°C. To minimize error due to the difference in bone mineral density of each test piece according to age, degree of growth and sex, the bone mineral density was measured (using dual energy X-ray absorptiometry, DPX-L, USA, made by Lunar) at the portion, where osteotomy would be performed, of the proximate portion of the tibia, and only the tibias belonging to a range of a certain bone mineral density (0.8 through 1.2mg/cm²) were used.

### (2) Staple used in the test

1) Coventry staple (made by Zimmer, USA)
   * material - stainless steel
   * particulars -
      length : 33mm
      width : 23.8mm
      offset : 9.5mm
2) Mini-plate staple of the present invention
   * material - ASTM F-90, Cobalt Chrome Alloy
   * particulars -
      length : 40mm
      width : 26mm
      offset : 8mm

### 2. Test Method

### (1) Preparation of test piece

To keep the porcine tibia in a fresh state before the test, it was refrigerated and kept in a refrigerator below -72°C, then thawed at room temperature for two hours before the test. The fibula and soft tissues around the tibia which do not affect the safety of the staple are removed right before the test so as to remove obstacles from the test portion. To facilitate the mounting of the tibia for test, distal portion of the tibia is cut out so as to make the total height of each test piece the same, 120mm.

### (A) Preparation of test piece for pull out test

To measure a simple holding power when an axial pull-out force is applied to the staple, one coventry staple (1 group : 5 pieces) or mini-plate staple is inserted in the same way as would be applied to a human body, external side, 20mm below a top end by utilizing 10 pieces of tibia on which the osteotomy was not performed, then the coventry staple or mini-plate staple is reinforced and fixed with two cortical screw of diameter 3.5mm (two groups : 5 pieces). To clamp the test piece in a material test system, 70mm of test piece bottom end is made as a rectangular cube and mounted by utilizing a resin, and at this time, to avoid error due to contamination of the resin, the staple inserting portion is coated with paraffin of sufficient quantity, and the reinforcement of the holding power by the resin is avoided.

### (B) Preparation of test piece for push out test

To provide the same condition as the osteotomy of the proximal portion of tibia carried out on the human body, the osteotomy is carried out at 20mm below the top end of tibia parallel to a flat portion of tibia by utilizing a vibration saw, and two kinds of staples are used in the same way as being applied to the human body, with 10 pieces of tibia divided into two groups each group having 5 pieces. To minimize the difference between tibias, test groups are formed by fixing different staples to the left and right tibia of one animal. Therefore, each of the left and right tibias of the animal belongs to different group. Group 1 is fixed by coventry staple and group 2 is fixed by mini-plate staple of the present invention, each group being composed of 5 tibias.

In the case of fixing with the mini-plate staple of the present invention, each 3.5mm cortical screw is inserted toward a forward and downward direction through a forward and backward hole of mini-plate attached to the lower part of the staple, the fitting is carried out after confirmation of whether the staple is fixed up to the distal end of the cortex.

### (C) Radiographical photographing

The depth, position and direction of insertion of the staple are confirmed by preparing a radiation ray photograph after manufacturing the test pieces of (A) and (B), and the test pieces which are not appropriate for the test method because of improper positioning of staple or improper direction of the bone fracture, are excluded from the test.

### (D) Fixing the test piece

The test piece is mounted in the shape of 60x60x70mm rectangular cube by using a resin to facilitate the fixing of the test piece to the material test system and to prevent direct transfer of the clamp force to the bone. If the resin is directly caught on the staple (especially, the entrance part), unnecessary reenforcement of holding power is caused, and to avoid this, the periphery of entrance of screw is coated with sufficient amount of paraffin before injection of the resin into container and the resin is filled only up to 1cm below the entrance of the staple so as to exclude the possibility of error in determining the holding power of the staple due to the resin. In addition, error due to rotational transformation is minimized by laterally inserting a screw of 10cm to tibial shaft prior to fitting and thereafter setting the resin.

### (2) Biomechanical test method

The test piece into which the staple is inserted is mounted on a fixed cross head of material test system (Autogram ET-5, made by Shimatzu, Japan), and a tension force is applied to a direction consistent to the axial direction of staple, then defining the maximum load at the moment when the staple is pulled out as the holding power of each staple, the load is measured as the value which is indicated on a chart recording instrument and a computer monitor and each test result is digitally stored into IBM 80386 computer by utilizing an interface data collection program (ASYST).

### a. Test I (pull out test)

The test piece is horizontally placed to the fixed cross head side of material test system, then the distal portion of tibia of test piece is fixed with the fixing piece so as to facilitate the axial tension of the staple. Then, a moving cross head is vertically moved at the velocity of 1mm/min, and a force-displacement curve up to the moment of the staple is pulled completely out of the bone is obtained for each test piece. At this time, defining the maximum load on the force-displacement curve as the pull-out strength due to the simple tension force of tested staple, the pull-out strength is recorded (Table 1).

### b. Test II ( push out test)

To provide a condition similar to a physiological load condition, the test piece is laterally placed to the fixed cross head side of material test system, then the distal portion of the test piece is fixed to the fixed cross head side with the fixing piece, fix and the proximal portion of test piece is placed to be pushed vertically downwards by the moving cross head. The proximal portion and distal portion of test piece will be separated eventually with the staple as a center according to the load increase due to a pressure of the moving cross head on the upper part of the proximal portion, and eventually the staple will be pushed out. The moving velocity of the moving cross head is taken as 1mm, and the force-displacement relation is measured until the moment of complete pushing out of the staple from the bone. At this time, defining the maximum load on the force-displacement curve as the push-out strength due to an axial force of tested staple, the push-out strength is recorded (Table 2).

### Results

### A) Test I, Pull-Out Strength

The pull-out strengths of coventry staple and mini-plate staple of the present invention are average 27.88±5.12kgf and 182.47±32.75kgf, respectively, the mini-plate being 6.54 times stronger (Table 1).

**Table 1.**

| Pull-out strength of coventry staple vs mini-plate staple | |
|---|---|
| coventry staple | Mini-plate staple |
| 23.750 | 196.250 |
| 25.000 | 223.750 |
| 28.875 | bone fracture |
| bone fracture | 158.000 |
| 33.875 | 181.875 |
| Average 27.88±5.12 | 182.47±32.75 |
| | (unit : kgf) |

### B) Test II, Push-Out Strength

The push-out strengths of the coventry staple and the mini-plate staple of the present invention average 18.40±4.47kgf and 119.95±19.06kg f, respectively, the mini-plate staple being 6.52 times stronger (Table 2).

**Table 2.**

| Push-out strength of coventry staple vs mini-plate staple | |
|---|---|
| coventry staple | Mini-plate staple |
| 22.875 | 116.120 |
| 16.000 | 129.000 |
| 17.875 | 109.250 |
| 18.000 | 122.370 |
| 17.250 | 123.000 |
| Average 18.40±4.47 | 119.95±19.16 |
| | (unit : kgf) |

Reviewing the tables shown above of the test, conclusions are drawn out as follows:
1. The pull-out strength of the mini-plate staple of the present invention is 6.54 times stronger than the coventry staple on the average.
2. The push-out strength of the mini-plate staple of the present invention is also 6.52 times stronger than the coventry staple on the average.

As a result of the above test, it can be appreciated that when the mini-plate staple of the present invention is clinically used after osteotomy on the proximal portion of tibia, since the fixing force of the mini-plate staple of the present invention is about 6 times stronger than the coventry staple, earlier movement of articulation is possible without the need of long term plaster fixing so that the time required for rehabilitation can be effectively shortened.

Although the mini-plate staple of the present invention is designed for the purpose of using in the osteotomy on the proximal portion of tibia, it can also be used for fixing the bone at different portions after various operations. That is, it can be used by inserting a fixing pin into each side of inner side and exterior side after osteotomy on a supracondylar of a femur, and thereafter fixing the pin with two screws.

The reason why the fixing force of the mini-plate of the present invention is more than 6 times stronger than the conventional staple is that although the fixing force of the conventional product is produced only by the fixing pin, the mini-plate staple uses two screws through the attached small metal plate so that the fixing force is produced by the fixing pin and two screws, and in addition, since a little amount of osteolysis appears around the fixing pin after operation when used in human bone, the mini-plate staple never slips out or moves after insertion compared to the conventional product in which only the fixing pin is used.

The mini-plate staple of the present invention formed as described above has advantages in that it does not need plaster fixing even after the operation, the movement of the articulation is possible right after the operation, walking with a crutch is possible in about 3 weeks after the operation, and no re-hospitalization for rehabilitation therapy is necessary.

## Claims

1. A plate-staple for use in osteotomy, said plate-staple comprising:
a fixing pin having a pair of legs with each leg terminating in a sharp point and with one of said pair of legs being longer relative to the other leg;
a bridging member for interconnecting said pair of legs of said fixing pin; and
a plate having first and second holes formed therein with a top recess formed between said first and second holes, such that in use each said first and second holes receive a screw for securing said plate and said top recess receives therein a portion of said bridging member upon operatively positioning said fixing pin to provide support.

2. The plate-staple of claim 1 wherein said plate has an oblong shape.

3. The plate-staple of claim 1, wherein said bridging member includes an offset portion and an aligned portion, with said aligned portion being received in said recess of said plate.

4. A plate-staple for use in osteotomy, said plate-staple comprising:
a fixing pin having a pair of legs with each leg terminating in a sharp point and with one of said pair of legs being longer relative to the other leg;
a bridging member for interconnecting said pair of legs of said fixing pin, wherein said bridging member includes an offset portion and an aligned portion, with said aligned portion being received in said recess of said plate; and
a plate having first and second holes formed therein with a top recess formed between said first and second holes, such that in use each said first and second holes receive a screw for securing said plate and said top recess receives therein a portion of said bridging member upon operatively positioning said fixing pin to provide support.
